# EUROPEAN PATENT APPLICATION

(11) **EP 4 368 215 A1**
(43) Date of publication of application: **15.05.2024**
(21) Application number: 23208612.4
(22) Date of filing: 08.11.2023
(51) Int. Cl.: A61L 27/42, A61L 27/46, A61L 27/54, A61L 27/58, B33Y 80/00

(54) **RESORBABLE DEVICE FOR BONE REGENERATION**

(30) Priority: 09.11.2022 IT 202200023112
(71) Applicant: D. & D. S.r.l., 15011 Acqui Terme (AL) (IT); Universita' degli Studi di Genova, 16126 Genova (IT)
(72) Inventor: DE ANGELIS, Nicola, I-15011 ACQUI TERME (Alessandria) (IT); BARBERIS, Fabrizio, I-15100 ALESSANDRIA (IT); LAGAZZO, Alberto, I-16011 ARENZANO (Genova) (IT); CIRILLO, Paolo, I-16030 AVEGNO (Genova) (IT); DE ANGELIS, Pasqualino, I-15011 ACQUI TERME (Alessandria) (IT)
(74) Representative: Aseglio-Gianinet, Romina

(57) **Abstract**

The present invention relates to a resorbable device for bone regeneration comprising a main body of bioresorbable polymeric material, wherein said main body comprises at least a portion for anchoring said device to a portion of alveolar bone, wherein said main body comprises at least an osteoconductive substance selected from: hydroxyapatite and/or tricalcium phosphate in alpha or beta form, brushite, monetite, or mixtures thereof.

The device according to the present invention, has the advantages of being an easy-to-graft bone regeneration device that does not require preoperative device modeling and does not require the removal thereof by means of a second surgical operation.

## Description

### Field of the invention

The present invention relates to a resorbable device for bone regeneration and a process for manufacturing such a device.

### Prior art

Evolving market trends currently make factors such as edentulism, dental defects, and issues related to both functional and aesthetic variables a major focus of the development lines of companies operating in the dental market. The causes of primary concern may be attributed essentially to two main factors:
1) the aging population and the need to create a system that is capable of ensuring dental function in all its aspects;
2) the demand for aesthetic factors that lead to the dental defects which were tolerated in years past no longer being considered socially acceptable.

The placement and grafting of a dental implant have become routine surgical procedures for dental professionals. During these procedures and in particular during the specific grafting phase, it is considered indispensable to graft the dental implant by guiding it within the three planes of space. Often, due to prior trauma or infection, the amount of available alveolar bone does not allow for proper grafting of the implant, this creates the necessity to provide a phase of reconstruction of the missing bone volume, prior to the dental implant grafting phase. The phase of reconstruction of the missing bone volume is usually performed by means of different techniques known to the person skilled in the art. They include: autologous bone grafts taken from other sites; use of devices made with biomaterials of synthetic or animal origin wrapped in resorbable membranes made of collagen, or non-resorbable membranes made of PTFE, or preformed titanium grids. Such grafts or devices have the disadvantage that, in order to be used, they require fixation to the bone defect with subsequent adaptation according to the morphology of the patient to be treated, which requires careful and laborious intraoperative processing in order to achieve the desired result.

This has some inevitable disadvantages, such as:
- the need to modify the graft or device to be grafted into the alveolar bone of the patient ex-ante, i.e., prior to surgery;
- the need to optimize the graft or the device once grafted in situ, i.e., during surgery;
- the increase in graft or device placement time and related surgical steps;
- the increased risks of infection during the various surgical steps;
- the increased discomfort imposed upon the patient during all surgical steps;
- the increased risk of a non-perfect match of the graft or device with the tissues of the patient.

Since 2015, "additive manufacturing" techniques have made it possible to obtain customized devices already at the design stage through the use of titanium alloy (Ti6Al4V), which, compared to devices made with the other technologies known to the person skilled in the art, allow for greater precision and time savings during the various surgical steps and for greater optimization of the functional properties of the device.

Titanium currently stands as the material used in about 70% of the products used, both as implants and as support structures.

However, these devices have the disadvantage that they require a second surgical operation to remove them when the device has fulfilled the function thereof in supporting bone regeneration. This second surgical procedure is, disadvantageously, often not easy to implement. It consists, in fact, in the laborious detachment of portions of the device from the regenerated bone, resulting in the possibility of fragments of the device being dispersed into the surrounding soft tissues, portions of the newly formed bone tissue being damaged, and the risks associated with secondary infections being increased.

In recent years, there is also a growing trend among patients to seek so-called "metal-free" grafts or devices in order to reduce possible causes of metal ion contamination and subsequent allergic reactions and/or metallosis.

In light of the above considerations, with the wide prevalence of the problem, and the extreme necessity to reduce the risks to both patients and surgeons who graft the aforesaid grafts or devices, there is a need to turn towards a medically optimized and publicly accessible solution at a reduced cost, resulting in a large potential for market development.

The possibility of having a bone regeneration device that is easy to graft, does not require a preoperative modeling of the device, and does not require the removal thereof by a second surgical procedure is therefore a need felt in the market.

### Summary of the invention

Therefore, the objective of the present invention is to provide a device for bone regeneration that is easy to graft, does not require preoperative modeling of the device, and does not require the removal thereof by a second surgery.

This is achieved by a resorbable device for bone regeneration and a process for manufacturing such a device as outlined in the attached claims, the definitions of which form an integral part of the present description.

### Brief description of the figures

The invention will be better understood from the following detailed description of preferred embodiments thereof, given by way of non-limiting example and with reference to the accompanying figure, wherein:

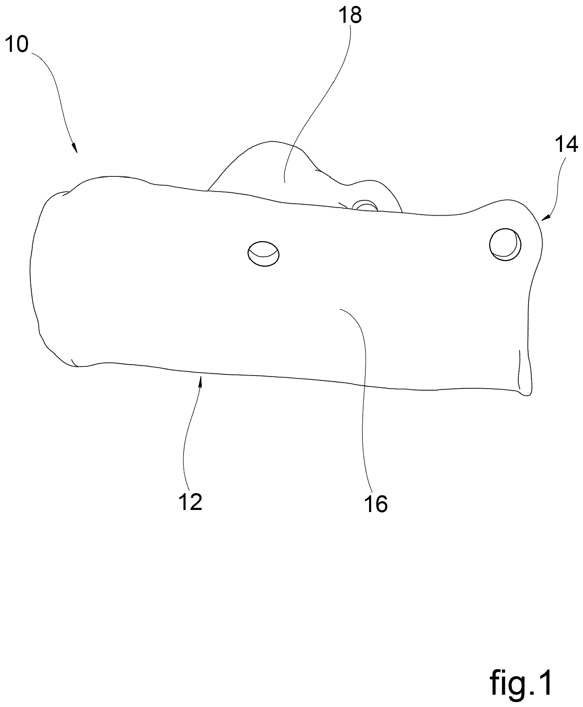

- Fig. 1 shows a side view of a device according to the present invention.

### Detailed description of the invention

With reference to Fig. 1, a first subject matter of the present invention is shown, namely, a resorbable device 10 for bone regeneration comprising a main body 12 of bioresorbable polymeric material, wherein said main body 12 comprises at least a portion 14 for anchoring said device to a portion of alveolar bone, wherein the main body 12 comprises at least an osteoconductive substance selected from: hydroxyapatite and/or tricalcium phosphate in alpha or beta form, brushite, monetite, or mixtures thereof.

Within the present description and related claims, the terms "resorbable" and "bioresorbable" refer to a device and/or material capable of being completely absorbed by the tissues of the patient on which the device is used.

Within the present description and related claims, the term "osteoconductive substance" refers to a substance that provides mechanical support to the blood vessels and cellular elements that will colonize the graft site.

According to a preferred embodiment of the device according to the present invention, in the main body 12 the at least an osteoconductive substance is preferably hydroxyapatite and tricalcium phosphate in beta form.

Also according to a preferred embodiment of the device according to the present invention, the main body 12 preferably has a solid curved foil or net structure. The term "solid structure" refers to a structure that for the majority of the configuration thereof provides for seamless construction and therefore is not continuously interrupted by holes or openings, as in the contrasting case of a net structure.

The main body 12 preferably has an irregular shape, wherein said main body 12, in addition to the anchor portion 14, preferably comprises at least a first unit 16 and at least a second unit 18 interconnected seamlessly or not, for example, using joining elements such as adhesive or fastening elements. Preferably at least one or both of the first unit 16 and the second unit 18 are connected either seamlessly or not to the anchorage portion 14. Preferably both the first unit 16 and the second unit 18 have a polygonal shape, even more preferably substantially parallelepiped.

According to a preferred embodiment of the device 10 according to the present invention, the anchor portion 14 also has preferably an irregular shape, even more preferably a polygonal shape, even more preferably a substantially parallelepiped shape. Preferably, said portion 14 comprises a hole or passage or slot or channel or opening, all regular or irregular, to accommodate or house at least an anchoring element such as a screw or bolt or rivet or rod or adhesive, for anchoring the portion 14 to a portion of alveolar bone.

According to a preferred embodiment, the portion 14 preferably comprises a hole for housing a screw that, passing through the hole, and penetrating into a portion of alveolar bone, anchors the portion 14 and thus the device 10 to the aforementioned portion of alveolar bone.

According to an alternative embodiment of the device 10 according to the present invention, the anchor portions 14 are preferably two, one connected to the first unit 16 and one connected to the second unit 18.

According to a preferred embodiment of the device 10 according to the present invention, the main body 12 preferably has a size such that it may easily be housed within the buccal cavity. Preferably it has a size of between 0. 1 cm² and 10 cm².

According to a preferred embodiment of the device 10 according to the present invention, the first unit 16 and the second unit 18 are spatially located therebetween at an angle of 1° to 90°.

According to an alternative embodiment of the device 10 according to the present invention, between the first unit 16 and the second unit 18 a third unit is comprised, placed substantially perpendicular to the units 16 and 18.

According to a preferred embodiment of the device 10 according to the present invention, the bioresorbable polymeric material preferably comprises at least one of: non-permanent-type ceramic-polymer compound, natural polymer, synthetic biodegradable polymer, or mixtures thereof.

Within the present description and the related claims, the term "ceramic-polymer compound" refers to a polymer compound that at the same time is also a ceramic compound, while the term "non-permanent type" refers to a compound that does not remain unchanged for an indefinite period of time but is absorbed by the tissues of the patient on which the device is used.

According to a preferred embodiment of the device 10 according to the present invention, the non-permanent ceramic-polymer compound is preferably selected from: aluminia, zirconia, or mixtures thereof, and/or the natural polymer is preferably selected from: alginate, chondroitin-6-sulfate, chitosan, hyaluronic acid, collagen, polylysine, dextran, heparin, or mixtures thereof, and/or the biodegradable synthetic polymer is preferably selected from: polyglycolic acid (PGA), polylactic acid (PLA) and the dextro and levo stereoisomers (PDLLA, PLLA) thereof, polyethylene oxide, poly(lactic-co-glycolic) acid (PLGA), polycaprolactone (PCL), polyanhydride, polyphosphazene, poly(ortho-ester), polyimide, poly(hydroxyalkanoate) (PHA), polybutene succinate (PBS), polybutyrate (PBAT), or mixtures thereof.

According to a preferred embodiment of the device 10 according to the present invention, the main body 12 further comprises preferably at least an osteoinductive substance.

Within the present description and related claims, the term "osteoinductive substance" refers to a substance that stimulates osteogenesis at the graft site.

Preferably, the at least an osteoinductive substance is selected from: cellular growth factors such as TGF, PDGF, LGF, FGF, morphogenetic proteins of the bone.

Within the present description and the related claims, the term "morphogenetic proteins of the bone" refers to a family of proteins involved in embryogenesis and specialized in the formation and growth of bone and cartilage.

A second subject matter of the present invention relates to a process for producing a resorbable device 10 for bone regeneration as described above, comprising the following steps:
a) providing at least a three-dimensional radiographic image of an alveolar bone defect;
b) importing the at least an image of step a) onto a 3D drawing program installed on an electronic computer;
c) using said 3D drawing program to measure sizes and volume of the alveolar bone defect;
d) using said 3D drawing program to draw a model of a device having the sizes and the volume of the alveolar bone defect of step c);
e) importing the model of the device of step d) into a 3D printing program that may be read by a 3D printer;
f) printing the device using the 3D printer of step e), thereby obtaining the resorbable device 10 for bone regeneration according to the present invention and as described above.

According to an embodiment of the process according to the present invention, in step f) the printing is preferably done using bioresorbable polymeric material comprising at least one of: non-permanent ceramic-polymer compound, natural polymer, biodegradable synthetic polymer, or mixtures thereof and preferably using at least an osteoconductive substance selected from: hydroxyapatite and/or tricalcium phosphate in alpha or beta form, brushite, monetite, or mixtures thereof.

Preferably, the non-permanent type ceramic-polymer compound is selected from: aluminia, zirconia, or mixtures thereof, and/or the natural polymer is selected from: alginate, chondroitin-6-sulfate, chitosan, hyaluronic acid, collagen, polylysine, dextran, heparin, or mixtures thereof, and/or the biodegradable synthetic polymer is selected from: polyglycolic acid (PGA), polylactic acid (PLA) and dextro and levo stereoisomers (PDLLA, PLLA) thereof, polyethylene oxide, poly(lactic-co-glycolic) acid (PLGA), polycaprolactone (PCL), polyanhydride, polyphosphazene, poly(ortho-ester), polyimide, poly(hydroxyalkanoate) (PHA), polybutene succinate (PBS), polybutyrate (PBAT), or mixtures thereof. Preferably the at least an osteoconductive substance is hydroxyapatite and tricalcium phosphate in beta form.

According to one embodiment of the process according to the present invention, in step f) the printing is preferably performed using, moreover, at least an osteoinductive substance. Preferably, the at least an osteoinductive substance is selected from: cellular growth factors such as TGF, PDGF, LGF, FGF, morphogenetic proteins of the bone.

The device according to the present invention is therefore advantageously a device adapted for replacing current devices in functionality, combining the following advantages:
- use of polymer material instead of metal alloys in order to reduce discomfort and side effects and to reduce production costs;
- use of 3D printer-printable polymer material in order to construct the device according to the present invention;
- use of resorbable polymeric material that is able to eliminate the need to perform a second surgery to remove the device at the end of the function thereof;
- use of resorbable polymeric material comprising osteoconductive substances (such as, but not limited to, hydroxyapatite) and possibly also with osteoinductive substances (such as, but not limited to, so-called "growth factors").

The device according to the present invention is thus, advantageously, a resorbable device that not only results in a reduction of the total cost of the surgical phase (only one surgical operation required and no longer two as with devices of the prior art) but also in a reduction of the so-called social costs borne by the patient and the production system (for example: reduction of the loss of work days), in addition to solving all the conditions of discomfort listed above.

Thus, the device according to the present invention is advantageously easy to graft, does not require preoperative modeling of the device, and does not require the removal thereof by means of a second surgery.

Since the device according to the present invention is a device made of bioresorbable polymeric material it is, advantageously, free of toxicity and harmful side effects, the feature of providing at least an osteoconductive substance also advantageously provides the ability to guide and cooperate in the process of bone regeneration, thus becoming an active part of the biological process of bone regeneration. An additional advantage is that it is a device that does not require removal with further surgery once the regenerative function thereof has been fulfilled.

Thus, the device according to the present invention has the following advantages:
1) the device need not be removed at the end of the treatment, thus avoiding future surgical complications, risks of infection, and reducing difficulties for the patient;
2) the bioresorbable polymeric material of the device according to the present invention, due to the presence of the osteoconductive substance, cooperates in the creation of new bone tissue in the osteoconductive (and optionally also osteoinductive) function thereof; the use of polymeric materials combining the polymer with osteoconductive substances (such as and not only hydroxyapatite) allows the device to become an integral part of the regenerative process, contributing to both the protective and new bone formation functions;
3) in accordance with the guidelines now in use, the device according to the present invention falls within the type of "metal-free" implants, thus reducing the contact and possible permanence of metal material in the patient's jawbone. In essence, the fact that the device is made of a bioresorbable polymeric material makes it possible, after being implanted, to avoid a second surgical removal procedure, which is instead necessary with currently available titanium alloy devices.

The device according to the present invention also has the advantage of having high mechanical strength.

Furthermore, the process for producing the device according to the present invention advantageously allows for obtaining customized devices for bone regeneration that do not require preoperative modeling of the devices. This is made possible by the fact that the polymer may be printed using a 3D printer and does not require more expensive equipment such as that used in additive manufacturing of titanium powders. Obtaining a resorbable device opens the market to wider use in the field of dentistry by virtue of greater accessibility and much more favorable ease of handling compared to the titanium devices currently present in the state of the art.

### EXAMPLE

An example of the production process of the device according to the present invention is now provided in the following, briefly and in a non-limiting manner:
after obtaining a three-dimensional radiographic image of the bone defect, the file in DICOM format is imported into a 3D drawing program where the operator virtually creates and positions the amount of bone that will have to be regenerated. After this step, the device that will be responsible for guiding and assisting the regeneration of the future bone is designed. The device thus generated is exported in STL format for 3D printing, which is performed using a bioresorbable polymeric material comprising an osteoconductive substance as the printing material.

## Claims

1. Resorbable device (10) for bone regeneration comprising a main body (12) of bioresorbable polymeric material, wherein said main body (12) comprises at least a portion (14) for anchoring said device to a portion of alveolar bone, wherein said main body (12) comprises at least an osteoconductive substance selected from: hydroxyapatite and/or tricalcium phosphate in alpha or beta form, brushite, monetite, or mixtures thereof.

2. Device according to claim 1, wherein in the main body (12) the at least an osteoconductive substance is hydroxyapatite and tricalcium phosphate in beta form.

3. Device according to claim 1 or 2, wherein the bioresorbable polymeric material comprises at least one of: non-permanent ceramic-polymer compound, natural polymer, biodegradable synthetic polymer, or mixtures thereof.

4. Device according to claim 3, wherein the non-permanent ceramic-polymer compound is selected from: aluminia, zirconia or mixtures thereof, and/or the natural polymer is selected from: alginate, chondroitin-6-sulfate, chitosan, hyaluronic acid, collagen, polylysine, dextran, heparin, or mixtures thereof, and/or the biodegradable synthetic polymer is selected from: polyglycolic acid (PGA), polylactic acid (PLA) and its dextro and levo stereoisomers (PDLLA, PLLA), polyethylene oxide, poly(lactic-co-glycolic) acid (PLGA), polycaprolactone (PCL), polyanhydride, polyphosphazene, poly(ortho-ester), polyimide, poly(hydroxyalkanoate) (PHA), polybutene succinate (PBS), polybutyrate (PBAT), or mixtures thereof.

5. Device according to any one of claims from 1 to 4, wherein the main body (12) further comprises at least an osteoinductive substance.

6. Device according to claim 5, wherein the at least an osteoinductive substance is selected from: cellular growth factors such as TGF, PDGF, LGF, FGF, bone morphogenetic proteins, or mixtures thereof.

7. Device according to any one of claims from 1 to 6, wherein the main body (12) has a solid curved foil structure or a net structure.

8. Process for producing a resorbable device (10) for bone regeneration according to any one of claims from 1 to 7, comprising the following steps:
a) providing at least a three-dimensional radiographic image of an alveolar bone defect;
b) importing the at least an image of step a) onto a 3D drawing program installed in an electronic computer;
c) using said 3D drawing program to measure sizes and volume of the alveolar bone defect;
d) using said 3D drawing program to draw a model of a device having the sizes and volume of the alveolar bone defect of step c)
e) importing the model of the device of step d) into a 3D printing program that may be read by a 3D printer;
f) printing the device using the 3D printer of step e), thereby obtaining the resorbable device (10) for bone regeneration according to any one of claims from 1 to 7.

9. Process according to claim 8, wherein in step f) the printing is performed using bioresorbable polymeric material comprising at least one of: non-permanent ceramic-polymer compound, natural polymer, biodegradable synthetic polymer, or mixtures thereof, and using at least an osteoconductive substance selected from: hydroxyapatite and/or tricalcium phosphate in alpha or beta form, brushite, monetite, or mixtures thereof.

10. Process according to claim 9, wherein in step f) the printing is performed using, furthermore, at least an osteoinductive substance.
